# EUROPEAN PATENT SPECIFICATION

(11) **EP 2 793 934 B1**
(45) Date of publication and mention of the grant of the patent: **23.08.2017**
(21) Application number: 12808263.3
(22) Date of filing: 21.12.2012
(51) Int. Cl.: A61K 38/48

(54) **NOVEL METHOD FOR THE MANUFACTURING OF DI-CHAIN PROTEINS FOR USE IN HUMANS**
NEUARTIGES VERFAHREN ZUR HERSTELLUNG VON DI-KETTEN-PROTEINEN ZUR VERWENDUNG BEI MENSCHEN
NOUVEAU PROCÉDÉ DE FABRICATION DES PROTÉINES DE CHAÎNE DI À UTILISER CHEZ L'HOMME

(30) Priority: 23.12.2011 EP 10010178; 23.12.2011 US 201161579771 P
(43) Date of publication of application: 29.10.2014
(73) Proprietor: Merz Pharma GmbH & Co. KGaA, 60318 Frankfurt am Main (DE)
(72) Inventor: GREIN, Swen, 64354 Reinheim (DE); HÖLSCHER, Kerstin, 61440 Oberursel (DE); EYLENSTEIN, Annett, 63571 Gellnhausen (DE)
(74) Representative: Ricker, Mathias
(86) International application number: PCT/EP2012/005361
(87) International publication number: WO 2013/091895

(56) References cited:
- WO-A2-2006/076902
- WO-A2-2008/008803
- US-A1- 2010 022 751

## Description

### FIELD OF THE INVENTION

This invention relates to a novel method for producing di-chain clostridial neurotoxins for use in humans from single-chain precursors. The method comprises the step of expressing a nucleic acid sequence encoding a single-chain precursor comprising a thrombin-cleavage site and the step of cleaving the single-chain precursor with a human thrombin, particularly a human thrombin drug product authorized for human therapeutic use. The invention further relates to novel di-chain clostridial neurotoxins and nucleic acid sequences encoding such novel di-chain clostridial neurotoxins.

### BACKGROUND OF THE INVENTION

Many natural proteins are di- or multimeric complexes of individual protein chains, which are either linked by non-covalent forces of by disulfide bonds between individual chains. In a number of cases, the individual protein chains of such di- or multimeric complexes are expressed together as a single-chain precursor molecule comprising the individual protein chains. The single-chain precursor molecule usually contains one or more cleavage signals for proteases, which post-translationally cleave the single-chain precursor molecule, so that the final di- or multimeric complex can form. In certain cases, the protease is a protease present in the expression host cell, in other cases, where the single-chain precursor molecule is the precursor of a protease, autocatalytic cleavage may occur if the respective cleavage site is present in the protein.

Examples of such di- or multimeric complexes include insulin, human proteases, such as thrombin, and bacterial toxins, such as Diphtheria toxin or clostridial toxins.

Clostridium is a genus of Gram-positive bacteria that includes important pathogens, such as *Clostridium botulinum* and *Clostridium tetani,* which produce neurotoxins such as botulinum toxin and tetanus toxin, respectively, which are among the strongest toxins known to man.

Both botulinum toxin and tetanus toxin act at several sites within the central nervous system. Botulinum toxin blocks the release of the neurotransmitter acetylcholine from axons at a neuromuscular junction, while tetanus toxin blocks inhibitory impulses by interfering with the release of neurotransmitters at the presynaptic junctions of inhibitory motor nerve endings.

In *Clostridium botulinum,* the botulinum toxin is formed as a protein complex comprising the neurotoxic component and non-toxic proteins, and complexes with either 300 kDa, 500 kDa or with 900 kDa are obtainable from cultures of *Clostridium botulinum.*

The neurotoxic component of botulinum toxin is a two-chain polypeptide with a molecular weight of approximately 150 kDa, with a 100 kDa heavy chain linked by a disulfide bond to a 50 kDa light chain. The heavy chain is responsible for entry into the neuronal cell, while the light chain comprises an endopeptidase activity responsible for cleaving one or more proteins that is/are part of the so-called SNARE-complex involved in the process resulting in the release of neurotransmitter into the synaptic cleft.

In recent years, Botulinum neurotoxins have become the standard agents in the treatment of focal dystonias and spastic indications. Preparations comprising botulinum toxin complexes are commercially available, e.g. from Ipsen Ltd. (Dysport^{®}) or Allergan Inc. (Botox^{®}). A high purity neurotoxic component, free of any complexing proteins, is for example available from Merz Pharmaceuticals GmbH, Frankfurt (Xeomin^{®}).

Treatment of patients generally involves injection of the neurotoxin, or neurotoxic component, into affected muscle tissue, bringing the agent near to the neuromuscular end plate, i.e. close to the cellular receptor mediating its uptake into the nerve cell controlling said affected muscle. Various degrees of neurotoxin spread have been observed. This spread is thought to correlate with the injected amounts and the particular preparation of neurotoxin injected. Resulting from the spread, systematic side effects caused by the inhibition of acetylcholine release, may be observed at nearby muscle tissue. The incidents of unintended paralysis of untreated muscles can largely be avoided by reducing the injected doses to the therapeutically relevant level. Overdosing may also be a problem with regard to the patients' immune system, as the injected neurotoxin may trigger the formation of neutralizing antibodies. If this occurs, the neurotoxin will be inactivated without being able to relieve the involuntary muscle activity.

Differences in the dose equivalents of preparations such as available sales products or batches produced during the manufacturing process, commonly a fermentation process, pose an increased risk for patients through possible side effects and the development of immunity. Therefore, it is of crucial importance to determine the biological activity of a clostridial neurotoxin contained in said sales products or production batches reliably (i.e. without significant variation) and as accurately as possible, in order to adjust the neurotoxin concentration to a reliable effective dose for the benefit of the patient. This may also serve as an incentive to the manufacturers to offer formulations allowing optimum exploitation of biological activity for different therapeutic purposes.

At present, clostridial neurotoxins are still predominantly produced by fermentation processes using appropriate Clostridium strains. Methods for the fermentation process using *Clostridium botulinum* strains are well known in the art (see, for example, Siegel and Metzger, Appl. Environ. Microbiol. 38 (1979) 606-611; Siegel and Metzger, Appl. Environ. Microbiol. 40 (1980) 1023-1026).

Additionally, clostridial neurotoxins can be produced in heterologous cells, i.e. be produced recombinantly by expressing nucleic acid sequences encoding a neurotoxin in appropriate host cells. Methods for the recombinant expression of clostridial neurotoxin in *E. coli* are well known in the art (see, for example, WO 00/12728, WO 01/14570, or WO 2006/076902). In certain cases, the light and heavy chains are separately obtained, and then reconstituted in vitro (see WO 95/32738).

Furthermore, clostridial neurotoxins have been expressed in eukaryotic expression systems, such as in *Pichia pastoris, Pichia methanolica, S. cerevisiae,* insect cells and mammalian cells (see WO 2006/017749).

In all these expression systems, a proteolytic cleavage of the single chain neurotoxin precursor is required, either by host cell enzymes during fermentation, or by adding proteolytic enzymes to the raw protein material isolated after fermentation, in order to generate the final biologically active clostridial neurotoxin protein comprising a light chain and a heavy chain linked by a disulfide bond.

In certain applications of recombinant expression, modified protease cleavage sites have been introduced recombinantly into the interchain region between the light and heavy chain of clostridial toxins, e.g. protease cleavage sites for non-human proteases (see WO 01/14570) or a protease site from BoNT/A into a BoNT/E construct (see WO 2009/014854).

In WO 2006/017749, the native loop region of Botulinum neurotoxins was modified by inserting a pentapeptide sequence motif VPXGS, particularly a pentapeptide sequence selected from VPRGS, VPHGS, VPYGS and VPQGS. After expression of modified variants of BoNT/A, BoNT/B and BoNT/C1 in *E. coli,* it was observed that in all cases more than 70% of the protein products were obtained in disulfide-linked di-chain form. Apparently, an unidentified *E. coli* protease must have cleaved the single-chain precursor product during fermentation and/or work-up. In the case of the modified BoNT/A product, it could be shown that the remaining single-chain product could be cleaved by using thrombin. The thrombin source was not mentioned in WO 2006/017749. It could furthermore be shown that the proteolytic cleavage in *E. coli* took place N-terminally from the newly introduced pentapeptide sequence between a lysine residue and the sequence SL ***VPXGS***, while treatment with thrombin is expected to occur between X and G in ***VPX-GS.*** Thus, such an approach of having a combination of an intrinsic *E. coli* proteolysis step and a thrombin cleavage step during work-up of the expression products inevitably creates a mixture of products, characterized by different termini at the C-terminus of the light chain and the N-terminus of the heavy chain.

Since clostridial toxins are being used for administration to humans, and in light of their high toxicity, there is a strong demand to produce the toxins with the highest possible purity and reproducibility, using materials that are fully compatible with the use in humans. So far, this aspect has not been solved satisfactorily.

### OBJECTS OF THE INVENTION

It was an object of the invention to improve the methods of the prior art and to develop a reliable and accurate method for manufacturing and obtaining di-chain proteins, such as clostridial neurotoxins, with high purity using components that are compatible with the use of such di-chain proteins in humans. In particular, a highly effective, i. e. near-complete cleavage of the precursor single-chain protein at a defined cleavage site, i. e. without accidental cleavage at other sites, particularly during expression in *E. coli*, is intended by the invention. Such a method and novel precursor proteins, such as clostridial neurotoxins precursors, used in such method would also serve to satisfy the great need for a safe and effective administration.

### SUMMARY OF THE INVENTION

Surprisingly it has been found that biologically active di-chain proteins, such as clostridial neurotoxins, for use in humans can be obtained recombinantly in functional form after expression from recombinant host cells by cloning a cleavage site for human thrombin into a gene encoding the single-chain precursor molecule and by cleavage with either human factor Xa or human thrombin, particularly with human thrombin-containing drug product authorized for human therapeutic use. Additionally, certain single-chain precursor molecules were surprisingly found to be not cleaved by *E. coli* proteases.

Described herein is a method for the generation of a disulfide-linked di-chain protein, comprising the step of (i) treating a disulfide-linked single-chain precursor protein, which comprises a cleavage signal for human thrombin in a linking peptide linking the C-terminus of a first chain with the N-terminus of a second protein chain, with a human factor Xa or a human thrombin.

Particularly, the human factor Xa is recombinant human factor Xa.

In one aspect, the present invention relates to a method for the generation of a disulfide-linked di-chain clostridial neurotoxin, comprising the step of treating a disulfide-linked single-chain precursor clostridial neurotoxin molecule, which comprises a cleavage signal for human thrombin in the loop region linking the C-terminus of the light chain with the N-terminus of the heavy chain, wherein the loop region linking the C-terminus of the light chain with the N-terminus of the heavy chain of the clostridial neurotoxin comprises the polypeptide motif ENKSLVPRGS, with human thrombin.

In certain embodiments, the human thrombin is recombinant human thrombin.

- In certain embodiments, the human thrombin is a human thrombin-containing drug product authorized for human therapeutic use.

In certain embodiments, the human thrombin-containing drug product is selected from Recothrom® and Evicel®.

Described herein are thrombin cleavage signals comprising a tripeptide motif Xxx-Arg-Yyy or Xxx-Lys-Yyy, preferably Pro-Arg-Yyy or Pro-Lys-Yyy, wherein Xxx is selected from Pro, Gly, Ala, Leu and Val, and Yyy is independently selected from Ser, Ala, Gly, Thr, Arg and Leu.

Described herein is a thrombin cleavage signal comprising the tetrapeptide motif Pro-Arg-Gly-Ser, preferably in the form of Leu-Val-Pro-Arg-Gly-Ser.

In particular embodiments, step (i) is performed with crude host cell lysates containing said single-chain precursor clostridial neurotoxin or with partially purified single-chain precursor clostridial neurotoxin, particularly after a first chromatographic enrichment step.

In a particular embodiment, step (i) is performed after a first chromatographic enrichment step, e. g. using Capto MMC (GE Healthcare) multimodal chromatography matrix.

In particular embodiments, where step (i) is performed with crude host cell lysates containing said single-chain precursor clostridial neurotoxin, between 2.5 and 900 international units (IU) of said human thrombin, particularly between 10 and 500 IU, more particularly between 50 and 300 IU, are used per mg of soluble total protein in the crude host cell lysate.

In particular embodiments, where step (i) is performed with partially purified single-chain precursor clostridial neurotoxin, particularly after a first chromatographic enrichment step, between 1 and 500 international units (IU) of said human thrombin, particularly between 5 and 200 IU, more particularly between 10 and 40 IU, are used per mg of single-chain precursor protein in the partially purified fraction.

In particular embodiments, step (i) is performed at a temperature between about 0°C and about 40°C.

In particular embodiments, step (i) is performed for between about 1 minute and about 24 hours.

In a particular embodiments, step (i) is performed at a temperature between about 0°C and about 15°C, particularly between about 0°C and about 12°C.

In particular such embodiments, step (i) is performed for between about 1 hour and 24 hours, particularly for between about 2 hours and about 18 hours, more particularly for 14 ± 2 hours.

In a particular embodiment step (i) is performed at a temperature between about 0°C and about 12°C for between about 20 min and about 5 h.

Surprisingly it has been found that recombinant single-chain clostridial neurotoxins, such as BoNT/E, containing a cleavage site for human thrombin, can be effectively cleaved with human thrombin-containing drug product authorized for human therapeutic use by incubation between about 20 min and about 5 h at a temperature between about 0°C and about 12°C.

In another embodiment, step (i) is performed at a temperature between about 15°C and about 40°C, particularly between about 15°C and about 30°C, more particularly at 20°C ± 2°C.

In particular such embodiments, step (i) is performed for between about 1 minute and about 2 hours, particularly for between about 5 minutes and about 1 hour, more particularly for between about 5 and about 20 minutes.

In particular embodiments, step (i) is performed in a buffer solution selected from the list of:
(i) Tris pH 7.6-8.0 and 70-150 mM NaCl;
(ii) Tris pH 7.6-8.0 and 300-500 mM NaCl;
(iii) Tris pH 7.8-8.2 and 20 mM NaCl;
(iv) Phosphate buffer pH 7.6-8.0 and 70-150 mM NaCl;
(v) Pphosphate buffer pH 7.6-8.0 and 300-500 mM NaCl;
(vi) Phosphate buffer pH 7.6-8.2 and 20 mM NaCl;
(vii) HEPES pH 7.6-8.0 and 70-150 mM NaCl;
(viii) HEPES pH 7.6-8.0 and 300-500 mM NaCl; and
(ix) HEPES pH 7.6-8.2 and 20 mM NaCl.

In particular embodiments, the buffer substance is present at a concentration of between about 10 mmol/l and about 100 mmol/l, particularly of between about 30 mol/l and about 70 mmol/l, more particularly of about 50 mmol/l.

In particular embodiments, step (i) is performed at a pH value of between about 6.8 and about 8.8, particularly between about 7.6 and about 8.0, more particularly at about 7.8.

In particular embodiments, the clostridial neurotoxin is selected from Clostridium botulinum neurotoxin serotype A, B, C, D, E, F, and G, particularly Clostridium botulinum neurotoxin serotype A and E, particularly Clostridium botulinum neurotoxin serotype E, more particularly a modified Clostridium botulinum neurotoxin serotype E.

In particular embodiments, the method further comprises the step of
(ii) isolating the disulfide-linked di-chain clostridial neurotoxin from the human thrombin-containing drug product by chromatography on an ion exchange matrix, a hydrophobic interaction matrix or a multimodal chromatography matrix, particularly a strong ion exchange matrix, more particularly a strong cation exchange matrix.

In a particular embodiments, step (ii) comprises one or more of the step(s):
(iia) a conditioning step to obtain a low-salt (≤ about 20 mM salt) solution at a pH of between about 7.9 and about 8.1, e.g. by appropriate dilution, diafiltration or dialysis;
(iib) application of said low-salt solution containing the disulfide-linked di-chain clostridial neurotoxin on a sulfopropyl-substituted chromatography matrix;
(iic) washing the sulfopropyl-substituted chromatography matrix with a buffer solution at a pH of between about 7.9 and about 8.1 and containing about 20 mM salt (e. g. NaCl)
(iid) elution of the disulfide-linked di-chain clostridial neurotoxin from the sulfopropyl-substituted chromatography matrix by increasing the salt concentration / ionic strength of the buffer solution of step (iic) to between about 50 and about 500 mM salt.

Described herein is a single-chain precursor molecule of a disulfide-linked di-chain protein comprising (i) a functionally active first chain, (ii) a functionally active second chain, and (iii) a loop region linking the first and the second chain, wherein the loop region comprises a cleavage signal for human thrombin.

Described herein is a single-chain precursor clostridial neurotoxin molecule has the sequence as found in SEQ ID NO: 6.

In another aspect, the present invention relates to a single-chain precursor clostridial neurotoxin molecule comprising (i) a functionally active clostridial neurotoxin light chain, (ii) a functionally active clostridial neurotoxin heavy chain, and (iii) a loop region linking the C-terminus of the light chain with the N-terminus of the heavy chain comprises a cleavage signal for human thrombin, wherein the loop region linking the C-terminus of the light chain with the N-terminus of the heavy chain of the clostridial neurotoxin comprises the polypeptide motif ENKSLVPRGS.

In another aspect, the present invention relates to a nucleic acid sequence encoding the single-chain precursor molecule of a clostridial neurotoxin molecule of the present invention.

Described herein is a nucleic acid sequence has the sequence as found in SEQ ID NO: 5.

In another aspect, the present invention relates to a method for obtaining the nucleic acid sequence of the present invention, comprising the step of inserting a cleavage signal for human thrombin into a nucleic acid sequence encoding the single-chain precursor molecule of a disulfide-linked di-chain clostridial neurotoxin molecule of the present invention.

In another aspect, the present invention relates to a vector comprising the nucleic acid sequence of the present invention, or the nucleic acid obtainable by the method of the present invention.

In another aspect, the present invention relates to a recombinant host cell comprising the nucleic acid sequence of the present invention, or the vector of the present invention.

In another aspect, the present invention relates to a method for producing the single-chain precursor molecule of a disulfide-linked di-chain clostridial neurotoxin molecule of the present invention, comprising the step of expressing the nucleic acid sequence of the present invention, the nucleic acid sequence obtainable by the method of the present invention, or the vector of the present invention in a recombinant host cell, or cultivating the recombinant host cell of the present invention under conditions that result in the expression of said nucleic acid sequence.

In particular embodiments, the method for producing the single-chain precursor molecule of a disulfide-linked di-chain clostridial neurotoxin molecule of the present invention, comprising the step of expressing the nucleic acid sequence of the present invention, the nucleic acid sequence obtainable by the method of the present invention, or the vector of the present invention in a recombinant host cell, or cultivating the recombinant host cell of the present invention under conditions that result in the expression of said nucleic acid sequence.

Described herein is a disulfide-linked di-chain protein, wherein the C-terminus of the first chain ends with the sequence VPR, particularly a sequence selected from KSLVPR and Xaa-Yaa-LVPR, wherein Xaa and Yaa are independently selected from the 20 naturally occurring amino acid residues, provided that the dipeptide sequence Xaa-Yaa is not K-S, particularly Xaa-Yaa-LVPR, particularly selected from TSLVP and GGLVP, and the N-terminus of the second chain begins with the sequence GSK.

In another aspect, the present invention relates to a disulfide-linked di-chain clostridial neurotoxin, wherein the C-terminus of the light chain ends with the sequence KSLVPR, and the N-terminus of the heavy chain begins with the sequence GSK.

In another aspect, the present invention relates to a pharmaceutical composition comprising the disulfide-linked di-chain clostridial neurotoxin of the present invention.

In particular embodiments, the pharmaceutical composition comprises a disulfide-linked di-chain clostridial neurotoxin of the present invention, and is for use in the treatment of a disease or condition taken from the list of: cervical dystonia (spasmodic torticollis), blepharospasm, severe primary axillary hyperhidrosis, achalasia, lower back pain, benign prostate hypertrophy, chronic focal painful neuropathies, migraine and other headache disorders, and cosmetic or aesthetic applications.

Described herein is a method of treating a disease or condition taken from the list of: cervical dystonia (spasmodic torticollis), blepharospasm, severe primary axillary hyperhidrosis, achalasia, lower back pain, benign prostate hypertrophy, chronic focal painful neuropathies, migraine and other headache disorders, and cosmetic or aesthetic applications; comprising the administration of the disulfide-linked di-chain clostridial neurotoxin of the present invention, or a pharmaceutical composition comprising a disulfide-linked di-chain clostridial neurotoxin of the present invention.

### DETAILED DESCRIPTION OF THE INVENTION

The present invention may be understood more readily by reference to the following detailed description of the invention and the examples included therein.

Described herein is a method for the generation of a disulfide-linked di-chain protein, comprising the step of (i) treating a disulfide-linked single-chain precursor protein, which comprises a cleavage signal for human thrombin in a linking peptide linking the C-terminus of a first chain with the N-terminus of a second protein chain, with a human factor Xa or a human thrombin.

In one aspect, the present invention relates to a method for the generation of a disulfide-linked di-chain clostridial neurotoxin, comprising the step of treating a disulfide-linked single-chain precursor clostridial neurotoxin molecule, which comprises a cleavage signal for human thrombin in the loop region linking the C-terminus of the light chain with the N-terminus of the heavy chain, wherein the loop region linking the C-terminus of the light chain with the N-terminus of the heavy chain of the clostridial neurotoxin comprises the polypeptide motif ENKSLVPRGS, with human thrombin.

In the context of the present invention, the term "comprises" or "comprising" means "including, but not limited to". The term is intended to be open-ended, that specify the presence of any stated features, elements, integers, steps, or components, but do not preclude the presence or addition of one or more other features, elements, integers, steps, components, or groups thereof. The term "comprising" thus includes the more restrictive terms "consisting of" and "consisting essentially of'.

In the context of the present invention, the term "disulfide-linked di-chain protein" refers to a protein comprising at least two chains elements, where one end of the first chain element is linked to one end of the second chain element not via a peptide bond, but via a disulfide bond.

In the context of the present invention, the term "disulfide-linked single-chain precursor protein" refers a precursor for a disulfide-linked di-chain protein, where the ends of at least two chain elements are linked by a polypeptide linker.

In the context of the present invention, the term "cleavage signal for human thrombin" relates to a polypeptide sequences that is recognized and cleaved by human thrombin. Cleavage signals for human thrombin are well known to anyone of ordinary skill in the art (see, for example, Schilling & Overall, Nature Biotechnology 26 (2008) 685-694).

Human factor Xa is known to cleave proteins C-terminally of the arginine residue in the factor Xa recognition sequence IDGR or IEGR. However, in certain instances factor Xa may also cleave proteins having a thrombin recognition sequence. Surprisingly, it was found that factor Xa very effectively cleaves the disulfide-linked single-chain precursor proteins in the methods of the present invention.

In the context of the present invention, the term "human thrombin-containing drug product authorized for human therapeutic use" refers to a drug product that contains a human thrombin, which product has been approved by a regulatory authority for use as a therapeutic in humans. In particular embodiments, the regulatory authority is selected from the European Medicines Agency (EMA), the U.S. Food and Drug Administration (FDA), and the Japanese Pharmaceuticals and Medical Devices Agency (PMDA).

In particular embodiments, cleavage of the precursor single-chain clostridial neurotoxin at a defined cleavage site is near-complete.

In the context of the present invention the term "near-complete" is defined as more than about 95% cleavage, particularly more than about 97.5%, more particularly more than about 99% as determined by SDS-PAGE or reversed phase chromatography.

Thus, in particular embodiments of the method of the present invention, the precursor single-chain clostridial neurotoxin is cleaved at the defined cleavage site to more than about 97.5%, more particularly more than about 99% as determined by SDS-PAGE or reversed phase chromatography.

In particular embodiments, cleavage of the precursor single-chain clostridial neurotoxin at a defined cleavage site is without accidental cleavage at other sites.

In the context of the present invention the term "without accidental cleavage" is defined as less than about 1%, particularly less than about 0.1%, more particularly less than about 0.01% of cleavage products other than the desired di-chain protein resulting from cleavage of the precursor single-chain protein, as determined by liquid chromatography-mass spectrometry (LC-MS) or mass spectrometry, than those resulting from the defined and intended cleavage site.

Thus, in particular embodiments of the method of the present invention, less than about 1%, particularly less than about 0.1%, more particularly less than about 0.01% of cleavage products other than the desired di-chain protein are resulting from cleavage of the precursor single-chain protein, as determined by LC-MS or mass spectrometry, than those resulting from the defined and intended cleavage site.

In the context of the present invention, the term "clostridial neurotoxin" refers to a natural neurotoxin obtainable from bacteria of the class Clostridia, including Clostridium tetani and Clostridium botulinum, or to a neurotoxin obtainable from alternative sources, including from recombinant technologies or from genetic or chemical modification. Particularly, the clostridial neurotoxins have endopeptidase activity.

In the context of the present invention, the term "recombinant clostridial neurotoxin" refers to a composition comprising a clostridial neurotoxin, that is obtained by expression of the neurotoxin in a heterologous cell such as *E. coli,* and including, but not limited to, the raw material obtained from a fermentation process (supernatant, composition after cell lysis), a fraction comprising a clostridial neurotoxin obtained from separating the ingredients of such a raw material in a purification process, an isolated and essentially pure clostridial neurotoxin, and a formulation for pharmaceutical and/or aesthetic use comprising a clostridial neurotoxin and additionally pharmaceutically acceptable solvents and/or excipients.

In the context of the present invention, the term "functionally active" refers to the property of a recombinant clostridial neurotoxin to perform the biological functions of a naturally occurring Clostridium botulinum neurotoxin to at least about 50%, particularly to at least about 60%, to at least about 70%, to at least about 80%, and most particularly to at least about 90%, where the biological functions include, but are not limited to, association of light and heavy chain to form the two-chain neurotoxin protein, entry of the neurotoxin into a neuronal cell, release of the light chain from the two-chain neurotoxin, and endopeptidase activity of the light chain. Methods for determining a neurotoxic activity can be found, for example, in WO 95/32738, which describes the reconstitution of separately obtained light and heavy chains of tetanus toxin and botulinum toxin.

In the context of the present invention, the term "about" or "approximately" means within 20%, alternatively within 10%, including within 5% of a given value or range. Alternatively, especially in biological systems, the term "about" means within about a log (i.e., an order of magnitude), including within a factor of two of a given value.

In the context of the present invention, the term "clostridial neurotoxin light chain" refers to that part of a clostridial neurotoxin that comprises an endopeptidase activity responsible for cleaving one or more proteins that is/are part of the so-called SNARE-complex involved in the process resulting in the release of neurotransmitter into the synaptic cleft: In naturally occurring clostridial neurotoxins, the light chain has a molecular weight of approx. 50 kDa.

In the context of the present invention, the term "clostridial neurotoxin heavy chain" refers to refers to that part of a clostridial neurotoxin that is responsible for entry of the neurotoxin into the neuronal cell: In naturally occurring clostridial neurotoxins, the heavy chain has a molecular weight of approx. 100 kDa.

In certain embodiments, the human thrombin is recombinant human thrombin.

The use of recombinant human factor Xa and/or human thrombin provides the advantage that animal products, such as bovine serum albumin or the like can be avoided during production of the protease, which may simplify the regulatory path for producing pharmaceutical products using such proteases.

In certain embodiments, the human thrombin is a human thrombin-containing drug product authorized for human therapeutic use, or is a human thrombin-containing drug substance which is also contained in a drug product authorized for human therapeutic use.

In the context of the present invention, a human thrombin-containing drug product authorized for human therapeutic use is a human thrombin that is regarded as safe by regulatory authorities for use in the production of drug product authorized for human therapeutic use.

In certain embodiments, the human thrombin for a thrombin-containing drug product authorized for human therapeutic use is isolated from human plasma cells, produced according to GMP regulations, and/or is used as pharmaceutical agent in a human drug product used as a Fibrin sealant.

In certain embodiments, the human thrombin is manufactured according to the Good Manufacturing Practice (GMP) for human use.

In certain embodiments, the human thrombin-containing drug product is selected from Recothrom^{®} and Evicel^{®}. In other embodiments, the human thrombin-containing drug product is Beriplast® P.

Described herein are thrombin cleavage signals comprising a tripeptide motif Xxx-Arg-Yyy or Xxx-Lys-Yyy, preferably Pro-Arg-Yyy or Pro-Lys-Yyy, wherein Xxx is selected from Pro, Gly, Ala, Leu and Val, and Yyy is independently selected from Ser, Ala, Gly, Thr, Arg and Leu.

Described herein is a thrombin cleavage signal comprising the tetrapeptide motif Pro-Arg-Gly-Ser, preferably in the form of Leu-Val-Pro-Arg-Gly-Ser.

Described herein is a loop region linking the C-terminus of the light chain with the N-terminus of the heavy chain of a clostridial neurotoxin and comprising one of the polypeptide motifs
KSLVPRGS; or
NKSLVPRGS.

Surprisingly, it has been found that removal of the dipeptide motif Lys-Ser from the N-terminus of the thrombin cleavage signal sequence LVPRGS in the loop region linking the C-terminus of the light chain with the N-terminus of the heavy chain of a clostridial neurotoxin results in a highly effective, i. e. near-complete cleavage of the precursor single-chain protein at the defined cleavage site, i. e. without accidental cleavage at other sites. Accidental cleavage was particularly observed during expression of the precursor single-chain protein in *E. coli* host cells (see WO 2006/076902 as discussed in Section [0016] above).

Described herein is a loop region linking the C-terminus of the light chain with the N-terminus of the heavy chain of a clostridial neurotoxin which does not comprise the dipeptide motif Lys-Ser at the N-terminus of the thrombin cleavage signal sequence LVPRGS. Thus, the loop region linking the C-terminus of the light chain with the N-terminus of the heavy chain of a clostridial neurotoxin comprises a dipeptide motif Xaa-Yaa N-terminally of the thrombin cleavage signal sequence LVPRGS, wherein Xaa and Yaa are independently selected from the 20 naturally occurring amino acid residues, provided that the dipeptide sequence Xaa-Yaa is not K-S.

Described herein is a loop region comprising one of the polypeptide motifs TSLVPRGS or
GGLVPRGS.

In particular embodiments, between 1 and 1000 international units (IU) of said human thrombin-containing drug product, particularly between 5 and 500 IU, more particularly between 10 and 200 IU, are used per mg of said disulfide-linked single-chain precursor protein (as determined with the Bradford reagent, Sigma-Aldrich article no. B6916, according to the colorimetric method of Bradford (Bradford, M.M. (1976): A rapid and sensitive method for the quantitation of microgram quantities of protein utilizing the principle of protein-dye binding. Anal. Biochem. Vol. 72, 248-254)).

In particular embodiments, step (i) is performed with crude host cell lysates containing said single-chain precursor clostridial neurotoxin or with partially purified single-chain precursor clostridial neurotoxin, particularly after a first chromatographic enrichment step.

In particular embodiments, where step (i) is performed with crude host cell lysates containing said single-chain precursor clostridial neurotoxin, between 2.5 and 900 international units (IU) of said human thrombin, particularly between 10 and 500 IU, more particularly between 50 and 300 IU, are used per mg of soluble total protein in the crude host cell lysate.

In a particular embodiment, step (i) is performed after a first chromatographic enrichment step, particularly using a multimodal chromatography matrix, particularly Capto MMC (GE Healthcare).

In particular embodiments, where step (i) is performed with partially purified single-chain precursor clostridial neurotoxin, particularly after a first chromatographic enrichment step, between 1 and 500 international units (IU) of said human thrombin, particularly between 5 and 200 IU, more particularly between 10 and 40 IU, are used per mg of single-chain precursor protein in the partially purified fraction.

In particular embodiment, step (i) is performed at a temperature between about 0°C and about 15°C, particularly between 0°C and about 12°C.

In particular such embodiments, step (i) is performed for between about 1 hour and 24 hours, particularly for between about 2 hours and about 18 hours, more particularly for 14 ± 2 hours.

In a particular embodiment step (i) is performed at a temperature between about 0°C and about 12°C for between about 20 min and about 5 h.

In another embodiment, step (i) is performed at a temperature between about 15°C and about 40°C, particularly between about 15°C and about 30°C, more particularly at 20°C ± 2°C.

In particular such embodiments, step (i) is performed for between about 1 minute and about 2 hours, particularly for between about 5 minutes and about 1 hour, more particularly for between about 5 and about 20 minutes.

In particular embodiments, step (i) is performed in a buffer solution selected from the list of:
(i) Tris pH 7.6-8.0 and 70-150 mM NaCl;
(ii) Tris pH 7.6-8.0 and 300-500 mM NaCl;
(iii) Tris pH 7.8-8.2 and 20 mM NaCl;
(iv) Phosphate buffer pH 7.6-8.0 and 70-150 mM NaCl;
(v) Phosphate buffer pH 7.6-8.0 and 300-500 mM NaCl;
(vi) Phosphate buffer pH 7.6-8.0 and 20 mM NaCl;
(vii) HEPES pH 7.6-8.0 and 70-150 mM NaCl;
(viii) HEPES pH 7.6-8.0 and 300-500 mM NaCl; and
(ix) HEPES pH 7.6-8.2 and 20 mM NaCl.

In particular embodiments, the buffer substance is present at a concentration of between about 10 mmol/l and about 100 mmol/l, particularly of between about 30 mol/l and about 70 mmol/l, more particularly of about 50 mmol/l.

In particular embodiments, step (i) is performed at a pH value of between about 6.8 and about 8.8, particularly between about 7.6 and about 8.0, more particularly at about 7.8.

In particular embodiments, the clostridial neurotoxin is selected from Clostridium botulinum neurotoxin serotype A, B, C, D, E, F, and G, particularly Clostridium botulinum neurotoxin serotype A and E, particularly Clostridium botulinum neurotoxin serotype E, more particularly a modified Clostridium botulinum neurotoxin serotype E.

In one embodiment of the present invention, the recombinant clostridial neurotoxin is a functional homologue of a *Clostridium botulinum* neurotoxin taken from the list of: *Clostridium botulinum* neurotoxin serotype A, B, C, D, E, F, and G.

In the context of the present invention, the term "*Clostridium botulinum* neurotoxin serotype A, B, C, D, E, F, and G" refers to neurotoxins obtainable from *Clostridium botulinum.* Currently, seven serologically distinct types, designated serotypes A, B, C, D, E, F and G, are known, including certain subtypes (e.g. A1, A2, and A3).

In a particular embodiment, the recombinant clostridial neurotoxin is a functional homologue of a *Clostridium botulinum* neurotoxin serotype E.

In the context of the present invention, the term "functional homologue of a *Clostridium botulinum* neurotoxin" refers to a neurotoxin that differs in the amino acid sequence, or the nucleic acid sequence encoding the amino acid sequence, from a *Clostridium botulinum* neurotoxin but is still functionally active. On the protein level, a functional homologue will maintain key features of the corresponding *Clostridium botulinum* neurotoxin, such as key residues for the endopeptidase activity in the light chain, or for the attachment to the neurotoxin receptors in the heavy chain, but may contain one or more mutations comprising a deletion of one or more amino acids of the parental *Clostridium botulinum* neurotoxin, an addition of one or more amino acids to the parental *Clostridium botulinum* neurotoxin and/or a substitution of one or more amino acids of the parental *Clostridium botulinum* neurotoxin. Preferably, said deleted or added amino acids are consecutive amino acids. According to the teaching of the present invention, any number of amino acids may be added or deleted, as long as the neurotoxin is biologically active. For example, 1, 2, 3, 4, 5, up to 10, up to 15, up to 25, up to 50, up to 100, up to 200, up to 400, up to 500 amino acids or even more amino acids may be added or deleted. In certain aspects, the present invention refers to neurotoxins, with an addition of more than 500 amino acids, such as for example up to 600 or up to 800 additional amino acids, or even more additional amino acids. Accordingly, a derivative of the neurotoxin may be a biologically active fragment of a naturally occurring neurotoxin. This neurotoxin fragment may contain an N-terminal, C-terminal and/or one or more internal deletion(s). "Biologically active", as used in this context, means, said derivative is taken up into the nerve cell and is capable of denervating said nerve from the muscle or gland, to which it is connected. Methods for designing and constructing homologues of a *Clostridium botulinum* neurotoxin and for testing of such homologues for functionality are well known to anyone of ordinary skill in the art.

In particular embodiments, the functional homologue has a sequence identity of at least about 40%, at least about 50%, at least about 60%, at least about 70% or most particularly at least about 80%, and a sequence homology of at least about 60%, at least about 70%, at least about 80%, at least about 90%, or most particularly at least about 95%. Methods and algorithms for determining sequence identity and/or homology, including the comparison of variants having additions and/or insertions relative to a parental sequence, are well known to the practitioner of ordinary skill in the art. On the DNA level, the nucleic acid sequences encoding the functional homologue and the parental Clostridium neurotoxin may differ to a larger extent due to the degeneracy of the genetic code. It is known that the usage of codons is different between prokaryotic and eukaryotic organisms (see, for example, Table 1 in WO 2006/017749). Thus, when expressing a prokaryotic protein such as a Clostridium neurotoxin, in a eukaryotic expression system, it may be necessary, or at least helpful, to adapt the nucleic acid sequence to the codon usage of the expression host cell, meaning that sequence identity or homology may be rather low on the nucleic acid level.

In particular embodiments, the functional homologue of the Clostridium neurotoxin is a derivative of the Clostridium neurotoxin.

In the context of the present invention, the term "derivative" and the term "variant" or "synthetic analogue" each individually refer to a neurotoxin that is a chemically, enzymatically or genetically modified derivative of a parental Clostridium neurotoxin, including chemically or genetically modified neurotoxin from C. *botulinum,* particularly of C. *botulinum* neurotoxin serotype A. A chemically modified derivative may be one that is modified by pyruvation, phosphorylation, sulfatation, lipidation, pegylation, glycosylation and/or the chemical addition of an amino acid or a polypeptide comprising between 2 and about 100 amino acids, including modification occurring in the eukaryotic host cell used for expressing the derivative. An enzymatically modified derivative is one that is modified by the activity of enzymes, such as endo- or exoproteolytic enzymes, including by modification by enzymes of the eukaryotic host cell used for expressing the derivative. As pointed out above, a genetically modified derivative is one that has been modified by deletion or substitution of one or more amino acids contained in, or by addition of one or more amino acids (including polypeptides comprising between 2 and about 100 amino acids) to, the amino acid sequence of said Clostridium neurotoxin. Methods for making such chemically or genetically modified derivatives, and methods for identifying whether such derivatives maintain neurotoxic activity, are well known to anyone of ordinary skill in the art.

In particular embodiments, the method further comprises the step of
(ii) isolating the disulfide-linked di-chain clostridial neurotoxin from the human thrombin-containing drug product by chromatography on an ion exchange matrix or a hydrophobic interaction matrix or a multimodal chromatography matrix, particularly a strong ion exchange matrix, more particularly a strong cation exchange matrix.

In particular such embodiments, step (ii) comprises the step(s) of:
(iia) a conditioning step to obtain a low-salt (≤ about 20 mM salt) solution at a pH of between about 7.9 and about 8.1, e.g. by appropriate dilution, diafiltration or dialysis;
(iib) application of said low-salt solution containing the disulfide-linked di-chain clostridial neurotoxin on a sulfopropyl-substituted chromatography matrix;
(iic) washing the sulfopropyl-substituted chromatography matrix with a buffer solution at a pH of between about 7.9 and about 8.1 and containing about 20 mM salt (e. g. NaCl)
(iid) elution of the disulfide-linked di-chain clostridial neurotoxin from the sulfopropyl-substituted chromatography matrix by increasing the salt concentration / ionic strength of the buffer solution of step (iic) to between about 50 and about 500 mM salt.

Described herein is a single-chain precursor molecule of a disulfide-linked di-chain protein comprising (i) a functionally active first chain, (ii) a functionally active second chain, and (iii) a loop region linking the first and the second chain, wherein the loop region comprises a cleavage signal for human thrombin.

In another aspect, the present invention relates to a single-chain precursor clostridial neurotoxin molecule comprising (i) a functionally active clostridial neurotoxin light chain, (ii) a functionally active clostridial neurotoxin heavy chain, and (iii) a loop region linking the C-terminus of the light chain with the N-terminus of the heavy chain comprises a cleavage signal for human thrombin, wherein the loop region linking the C-terminus of the light chain with the N-terminus of the heavy chain of the clostridial neurotoxin comprises the polypeptide motif ENKSLVPRGS.

In a particular embodiment of the present invention, the light chain has the amino acid sequence as found in SEQ ID NO: 1.

In a particular embodiment of the present invention, the heavy chain has the amino acid sequence as found in SEQ ID NO: 2.

In a further particular embodiment of the present invention, the light chain has the amino acid sequence as found in SEQ ID NO: 1, and the heavy chain has the amino acid sequence as found in SEQ ID NO: 2.

In another embodiment of the present invention, the recombinant clostridial neurotoxin is a functional homologue of a *Clostridium tetani* neurotoxin.

In the context of the present invention, the term "functional homologue of a *Clostridium tetani* neurotoxin" refers to a neurotoxin that differs in the amino acid sequence, or the nucleic acid sequence encoding the amino acid sequence, from a *Clostridium tetani* neurotoxin but is still functionally active. On the protein level, a functional homologue will maintain key features of the corresponding *Clostridium tetani* neurotoxin, such as key residues for the endopeptidase activity in the light chain, or for the attachment to the neurotoxin receptors in the heavy chain, but may contain one or more mutations comprising a deletion of one or more amino acids of the parental *Clostridium tetani* neurotoxin, an addition of one or more amino acids to the parental *Clostridium tetani* neurotoxin and/or a substitution of one or more amino acids of the parental *Clostridium tetani* neurotoxin. Preferably, said deleted or added amino acids are consecutive amino acids. According to the teaching of the present invention, any number of amino acids may be added or deleted, as long as the neurotoxin is biologically active. For example, 1, 2, 3, 4, 5, up to 10, up to 15, up to 25, up to 50, up to 100, up to 200, up to 400, up to 500 amino acids or even more amino acids may be added or deleted. In certain aspects, the present invention refers to neurotoxins, with an addition of more than 500 amino acids, such as for example up to 600 or up to 800 additional amino acids, or even more additional amino acids. Accordingly, a derivative of the neurotoxin may be a biologically active fragment of a naturally occurring neurotoxin. This neurotoxin fragment may contain an N-terminal, C-terminal and/or one or more internal deletion(s). "Biologically active", as used in this context, means, said derivative is taken up into the nerve cell and is capable of denervating said nerve from the muscle or gland, to which it is connected. Methods for designing and constructing homologues of a *Clostridium tetani* neurotoxin and for testing of such homologues for functionality are well known to anyone of ordinary skill in the art.

In particular embodiments, the functional homologue has a sequence identity of at least about 40%, at least about 50%, at least about 60%, at least about 70% or most particularly at least about 80%, and a sequence homology of at least about 60%, at least about 70%, at least about 80%, at least about 90%, or most particularly at least about 95%. Methods and algorithms for determining sequence identity and/or homology, including the comparison of variants having additions and/or insertions relative to a parental sequence, are well known to the practitioner of ordinary skill in the art. On the DNA level, the nucleic acid sequences encoding the functional homologue and the parental Clostridium neurotoxin may differ to a larger extent due to the degeneracy of the genetic code. It is known that the usage of codons is different between prokaryotic and eukaryotic organisms (see, for example, Table 1 in WO 2006/017749). Thus, when expressing a prokaryotic protein such as a Clostridium neurotoxin, in a eukaryotic expression system, it may be necessary, or at least helpful, to adapt the nucleic acid sequence to the codon usage of the expression host cell, meaning that sequence identity or homology may be rather low on the nucleic acid level.

In a particular embodiment of the present invention, the light chain has the sequence as found in SEQ ID NO: 3, and the heavy chain has the sequence as found in SEQ ID NO: 4.

In another aspect, the present invention relates to a nucleic acid sequence encoding the single-chain precursor molecule of a disulfide-linked di-chain clostridial neurotoxin molecule of the present invention.

Described herein is a clostridial neurotoxin molecule, having the sequence as found in SEQ ID NO: 6.

In certain embodiments, the present invention relates to a nucleic acid sequence encoding the recombinant clostridial neurotoxin of the present invention, comprising (i) a nucleic acid sequence encoding a functionally active clostridial neurotoxin light chain, (ii) a nucleic acid sequence comprising a sequence encoding a human thrombin cleavage site, and (iii) a nucleic acid sequence encoding a functionally active clostridial neurotoxin heavy chain.

In particular embodiments, the nucleic acid sequences (i), (ii) and (iii) encode a single contiguous amino acid sequence.

In the context of the present invention, the term "single contiguous amino acid sequence" refers to an amino acid sequence that would, when expressed from the corresponding nucleic acid sequence in an appropriate host cell in the absence of protease activity, be formed as a single chain protein.

In a particular embodiment, the nucleic acid sequence has the sequence as found in SEQ ID NO: 5.

In another aspect, the present invention relates to a nucleic acid sequence encoding the recombinant clostridial neurotoxin of the present invention as described elsewhere herein in detail, wherein the coding sequence encoding said neurotoxin comprises a coding sequence for a signal peptide. Usually, said coding sequence encoding the signal peptide will be located upstream of the coding sequence encoding the neurotoxin. Many such signal sequences are known in the art and are comprised by the present invention. In particular, the signal sequence is a signal peptide resulting in transport of the neurotoxin across a biological membrane, such as the membrane of the endoplasmic reticulum, the golgi membrane or the plasma membrane of a eukaryotic or prokaryotic cell. It has been found that signal sequences, when attached to the neurotoxin, will mediate secretion of the neurotoxin into the supernatant of the cells. In certain embodiments, the signal peptide will be cleaved off in the course of, or subsequent to, such secretion, so that the secreted protein lacks the N-terminal signal peptide, is composed of separate light and a heavy chains, which are covalently linked by S-S bridges and which are proteolytically active.

In another aspect, the recombinant clostridial neurotoxin according to the present invention comprises an N-terminal elongation of 5 to 50 amino acid residues, said elongation comprising a signal peptide capable of mediating transport across a biological membrane. In a more specific aspect of the present invention, this signal peptide is removable by a protease.

In another aspect, the present invention relates to a method for obtaining the nucleic acid sequence of the present invention, comprising the step of inserting a cleavage signal for human thrombin into a nucleic acid sequence encoding the single-chain precursor molecule of a disulfide-linked di-chain clostridial neurotoxin molecule of the present invention.

In a particular embodiment, the insertion is achieved by inserting the cleavage signal for human thrombin into the nucleic acid sequence encoding wild-type clostridial neurotoxin. In another particular embodiment, the insertion results in the deletion and/or modification of one or more amino acid residues of said wild-type clostridial neurotoxin.

In another aspect, the present invention relates to a vector comprising the nucleic acid sequence of the present invention, or the nucleic acid obtainable by the method of the present invention.

A further aspect of the present invention relates to a recombinant host cell comprising a nucleic acid sequence or a vector of the present invention.

In another aspect, the present invention relates to a method for producing the single-chain precursor molecule of a disulfide-linked di-chain clostridial neurotoxin molecule of the present invention, comprising the step of expressing the nucleic acid sequence of the present invention, the nucleic acid sequence obtainable by the method of the present invention, or the vector of the present invention in a recombinant host cell, or cultivating the recombinant host cell of the present invention under conditions that result in the expression of said nucleic acid sequence.

Yet another aspect of the invention relates to a method for producing the single-chain precursor molecule of a disulfide-linked di-chain clostridial neurotoxin molecule of the present invention, comprising the step of expressing a nucleic acid sequence or vector of the present invention in a recombinant host cell, or cultivating a recombinant host cell of the present invention under conditions that result in the expression of the nucleic acid sequence.

Described herein is a disulfide-linked di-chain protein, wherein the C-terminus of the first chain ends with the sequence VPR, and the N-terminus of the second chain begins with the sequence GSK.

Described herein is a disulfide-linked di-chain clostridial neurotoxin, wherein the C-terminus of the light chain ends with the sequence VPR, particularly a sequence selected from KSLVPR and Xaa-Yaa-LVPR, wherein Xaa and Yaa are independently selected from the 20 naturally occurring amino acid residues, provided that the dipeptide sequence Xaa-Yaa is not K-S, particularly Xaa-Yaa-LVPR, particularly selected from TSLVP and GGLVP, and the N-terminus of the heavy chain begins with the sequence GSK.

In a further aspect, the present invention relates to a disulfide-linked di-chain clostridial neurotoxin, wherein the C-terminus of the light chain ends with the sequence KSLVPR, and the N-terminus of the heavy chain begins with the sequence GSK.

In a further aspect, the present invention relates to a pharmaceutical composition comprising the disulfide-linked di-chain clostridial neurotoxin of the present invention.

In one embodiment, the pharmaceutical composition comprises a disulfide-linked di-chain clostridial neurotoxin of the present invention, and is for use in the treatment of a disease or condition taken from the list of: cervical dystonia (spasmodic torticollis), blepharospasm, severe primary axillary hyperhidrosis, achalasia, lower back pain, benign prostate hypertrophy, chronic focal painful neuropathies, migraine and other headache disorders, and cosmetic or aesthetic applications.

Additional indications where treatment with Botulinum neurotoxins is currently under investigation and where the pharmaceutical composition of the present invention may be used, include pediatric incontinence, incontinence due to overactive bladder, and incontinence due to neurogenic bladder, anal fissure, spastic disorders associated with injury or disease of the central nervous system including trauma, stroke, multiple sclerosis, Parkinson's disease, or cerebral palsy, focal dystonias affecting the limbs, face, jaw, or vocal cords, TMJ pain disorders, diabetic neuropathy, wound healing, excessive salivation, vocal cord dysfunction, reduction of the Masseter muscle for decreasing the size of the lower jaw, treatment and prevention of chronic headache and chronic musculoskeletal pain, treatment of snoring noise, assistance in weight loss by increasing the gastric emptying time.

Described herein is a method of treating a disease or condition taken from the list of: cervical dystonia (spasmodic torticollis), blepharospasm, severe primary axillary hyperhidrosis, achalasia, lower back pain, benign prostate hypertrophy, chronic focal painful neuropathies, migraine and other headache disorders, and cosmetic or aesthetic applications; comprising the administration of a recombinant clostridial neurotoxin, or a pharmaceutical composition comprising a disulfide-linked di-chain clostridial neurotoxin, according to the present invention.

### EXAMPLES

### Comparative Example 1: Cloning and Expression of a recombinant Botulinum neurotoxin serotype E comprising a thrombin cleavage site

A first nucleic acid molecule encoding amino acids 1 to 412 of wild type Botulinum neurotoxin serotype E, followed by a modified Botulinum neurotoxin serotype E interchain region comprising a thrombin cleavage site (nucleotides 1-1308 of SEQ ID NO: 5) and with flanking restriction endonuclease sites suitable for cloning said nucleic acid molecule into a pMEx expression vector (proprietary vector, Merz Pharmaceuticals) is synthesized. A second nucleic acid molecule encoding amino acids 426 to 1252 of wild type Botulinum neurotoxin serotype E, preceded by a modified Botulinum neurotoxin serotype E interchain region comprising a thrombin cleavage site (nucleotides 1234-3789 of SEQ ID NO: 5) and with flanking restriction endonuclease sites suitable for cloning said nucleic acid molecule into a pMEx expression vector (proprietary vector, Merz Pharmaceuticals) is synthesized, wherein the restriction endonuclease site flanking the 5'end is identical to the restriction endonuclease site flanking the 3'end of the first DNA molecule. The first synthetic nucleic acid molecule is digested with restriction enzymes cutting the flanking restriction endonuclease sites. After digestion of the pMEx vector with the same restriction enzyme combination, followed by enzymatic dephosphorylation, the digested first synthetic nucleic acid molecule is directionally cloned into the linearized vector using a T4 DNA ligase reaction mix. The ligation mixture is transformed into chemically competent E. coli using a heat shock method, plated on Luria-Bertani agar plates containing 30 µg/ml Kanamycin, and placed in a 37°C incubator for overnight growth. Bacteria containing vector constructs are identified as Kanamycin-resistant colonies, of which several are used to inoculate separate small-scale liquid cultures containing 30 µg/ml Kanamycin, which are then incubated in a shaker at 37°C for overnight growth. After preparation of plasmid DNA using an alkaline lysis method, clones containing the desired plasmid construct pMEx-NTE-LC are identified by restriction analysis and subsequent DNA sequencing. The second nucleic acid molecule is digested with restriction enzymes cutting the flanking restriction endonuclease sites, and directionally cloned into pMEx-NTE-LC following the procedure described above. The resulting plasmid construct pMEx-NTE contains an open reading frame encoding, from N- to C-terminus, amino acids 1 to 412 of wild type Botulinum neurotoxin serotype E, followed by a modified Botulinum neurotoxin serotype E interchain region comprising a thrombin cleavage site, followed by amino acids 426 to 1252 of wild type Botulinum neurotoxin serotype E (SEQ ID NO: 5), so that transcription of the open reading frame from the tac promoter of the vector is enabled.

The expression of the modified BoNT/E is achieved as follows. The prokaryotic expression plasmid pMEx-NTE encoding Botulinum neurotoxin serotype E with a modified interchain region (SEQ ID NO: 6) is introduced into chemically competent bacterial cells suitable for expression of the pMEx-NTE expression construct using a standard transformation protocol, such as, e.g., a heat-shock transformation protocol. The transformation reactions are plated onto Luria-Bertani agar plates containing suitable antibiotics and placed in a 37°C incubator for overnight growth. A single antibiotic-resistant colony of transformed cells containing pMEx-NTE is used to inoculate a 10 mL tube containing 5 mL Luria-Bertani medium supplemented with 30 µg/ml Kanamycin that is then placed in a 37 °C incubator, shaking at 250 rpm, for overnight growth. Approximately 3 mL of the resulting overnight starter culture is used to inoculate a 2000 mL baffled culture flask containing 800 mL Luria-Bertani medium supplemented with 30 µg/ml Kanamycin that is then placed in a 37 °C incubator, shaking at 250 rpm, for approximately 4-5 hours. After inducing the expression of the modified BoNT/E protein by adding IPTG to a final concentration of 0.5-1.0 mM, the culture is transferred to a 18°C incubator shaking at 170 rpm for overnight expression. Cells are harvested by centrifugation (4,000 rpm at 4 °C tor 20-30 minutes), and the pellet is resuspended in 200 mM Tris buffer pH 7.5 containing 100 mM NaCl. After cell lysis by high pressure homogenization at 2000 bar, the insoluble fraction is removed by centrifugation (20 min at 12000 x g, 4 °C) or by crossflow filtration using a 0,1 µm membrane. Culture samples taken before IPTG induction, at various time-points after induction, and after cell harvest are used to evaluate expression by SDS polyacrylamide gel electrophoresis and Western blot analysis using anti-BoNT/E antibodies.
**SEQ ID NO: 1:** 
1 MPKINSFNYN DPVNDRTILY IKPGGCQEFY KSFNIMKNIW IIPERNVIGT
51 TPQDFHPPTS LKNGDSSYYD PNYLQSDEEK DRFLKIVTKI FNRINNNLSG
101 GILLEELSKA NPYLGNDNTP DNQFHIGDAS AVEIKFSNGS QDILLPNVII
151 MGAEPDLFET NSSNISLRNN YMPSNHGFGS IAIVTFSPEY SFRFNDNSMN
201 EFIQDPALTL MHELIHSLHG LYGAKGITTK YTITQKQNPL ITNIRGTNIE
251 EFLTFGGTDL NIITSAQSND IYTNLLADYK KIASKLSKVQ VSNPLLNPYK
301 DVFEAKYGLD KDASGIYSVN INKFNDIFKK LYSFTEFDLA TKFQVKCRQT
351 YIGQYKYFKL SNLLNDSIYN ISEGYNINNL KVNFRGQNAN LNPRIITPIT
401 GRGLVKKIIR FCKNIVSVKG IR
**SEQ ID NO: 2:** 
423 KSICIEIN NGELFFVASE NSYNDDNINT
451 PKEIDDTVTS NNNYENDLDQ VILNFNSESA PGLSDEKLNL TIQNDAYIPK
501 YDSNGTSDIE QHDVNELNVF FYLDAQKVPE GENNVNLTSS IDTALLEQPK
551 IYTFFSSEFI NNVNKPVQAA LFVSWIQQVL VDFTTEANQK STVDKIADIS
601 IVVPYIGLAL NIGNEAQKGN FKDALELLGA GILLEFEPEL LIPTILVFTI
651 KSFLGSSDNK NKVIKAINNA LKERDEKWKE VYSFIVSNWM TKINTQFNKR
701 KEQMYQALQN QVNAIKTIIE SKYNSYTLEE KNELTNKYDI KQIENELNQK
751 VSIAMNNIDR FLTESSISYL MKLINEVKIN KLREYDENVK TYLLNYIIQH
801 GSILGESQQE LNSMVTDTLN NSIPFKLSSY TDDKILISYF NKFFKRIKSS
851 SVLNMRYKND KYVDTSGYDS NININGDVYK YPTNKNQFGI YNDKLSEVNI
901 SQNDYIIYDN KYKNFSISFW VRIPNYDNKI VNVNNEYTII NCMRDNNSGW
951 KVSLNHNEII WTLQDNAGIN QKLAFNYGNA NGISDYINKW IFVTITNDRL
1001 GDSKLYINGN LIDQKSILNL GNIHVSDNIL FKIVNCSYTR YIGIRYFNIF
1051 DKELDETEIQ TLYSNEPNTN ILKDFWGNYL LYDKEYYLLN VLKPNNFIDR
1101 RKDSTLSINN IRSTILLANR LYSGIKVKIQ RVNNSSTNDN LVRKNDQVYI
1151 NFVASKTHLF PLYADTATTN KEKTIKISSS GNRFNQVVVM NSVGNNCTMN
1201 FKNNNGNNIG LLGFKADTVV ASTWYYTHMR DHTNSNGCFW NFISEEHGWQ
1251 EK
**SEQ ID NO: 3:** 1 MPKINSFNYN DPVNDRTILY IKPGGCQEFY KSFNIMKNIW IIPERNVIGT 
51 TPQDFHPPTS LKNGDSSYYD PNYLQSDEEK DRFLKIVTKI FNRINNNLSG
101 GILLEELSKA NPYLGNDNTP DNQFHIGDAS AVEIKFSNGS QDILLPNVII
151 MGAEPDLFET NSSNISLRNN YMPSNHGFGS IAIVTFSPEY SFRFNDNSMN
201 EFIQDPALTL MHELIHSLHG LYGAKGITTK YTITQKQNPL ITNIRGTNIE
251 EFLTFGGTDL NIITSAQSND IYTNLLADYK KIASKLSKVQ VSNPLLNPYK
301 DVFEAKYGLD KDASGIYSVN INKFNDIFKK LYSFTEFDLA TKFQVKCRQT
351 YIGQYKYFKL SNLLNDSIYN ISEGYNINNL KVNFRGQNAN LNPRIITPIT
401 GRGLVKKIIR FCVRGIITSK TKSLVPR
**SEQ ID NO: 4:** 
428 GSK ALNDLCIEIN NGELFFVASE
451 NSYNDDNINT PKEIDDTVTS NNNYENDLDQ VILNFNSESA PGLSDEKLNL
501 TIQNDAYIPK YDSNGTSDIE QHDVNELNVF FYLDAQKVPE GENNVNLTSS
551 IDTALLEQPK IYTFFSSEFI NNVNKPVQAA LFVSWIQQVL VDFTTEANQK
601 STVDKIADIS IVVPYIGLAL NIGNEAQKGN FKDALELLGA GILLEFEPEL
651 LIPTILVFTI KSFLGSSDNK NKVIKAINNA LKERDEKWKE VYSFIVSNWM
701 TKINTQFNKR KEQMYQALQN QVNAIKTIIE SKYNSYTLEE KNELTNKYDI
751 KQIENELNQK VSIAMNNIDR FLTESSISYL MKLINEVKIN KLREYDENVK
801 TYLLNYIIQH GSILGESQQE LNSMVTDTLN NSIPFKLSSY TDDKILISYF
851 NKFFKRIKSS SVLNMRYKND KYVDTSGYDS NININGDVYK YPTNKNQFGI
901 YNDKLSEVNI SQNDYIIYDN KYKNFSISFW VRIPNYDNKI VNVNNEYTII
951 NCMRDNNSGW KVSLNHNEII WTLQDNAGIN QKLAFNYGNA NGISDYINKW
1001 IFVTITNDRL GDSKLYINGN LIDQKSILNL GNIHVSDNIL FKIVNCSYTR
1051 YIGIRYFNIF DKELDETEIQ TLYSNEPNTN ILKDFWGNYL LYDKEYYLLN
1101 VLKPNNFIDR RKDSTLSINN IRSTILLANR LYSGIKVKIQ RVNNSSTNDN
1151 LVRKNDQVYI NFVASKTHLF PLYADTATTN KEKTIKISSS GNRFNQVVVM
1201 NSVGNNCTMN FKNNNGNNIG LLGFKADTVV ASTWYYTHMR DHTNSNGCFW
1251 NFISEEHGWQ EK
**SEQ ID NO: 5:** 
1 ATGCCGAAAA TCAACAGCTT CAACTATAAC GATCCGGTGA ACGATCGTAC CATCCTGTAT
61 ATTAAACCGG GCGGTTGCCA GGAATTTTAC AAAAGCTTCA ACATCATGAA AAACATCTGG
121 ATTATTCCGG AACGTAACGT GATTGGCACC ACCCCGCAGG ATTTTCATCC GCCGACCAGC
181 CTGAAAAACG GCGATAGCAG CTATTATGAT CCGAACTATC TGCAGTCTGA TGAAGAAAAA
241 GATCGCTTCC TGAAAATCGT GACCAAAATC TTCAACCGCA TCAACAACAA CCTGAGCGGC
301 GGCATTCTGC TGGAAGAACT GAGCAAAGCG AATCCGTATC TGGGCAACGA TAACACTCCA
361 GATAACCAGT TTCATATTGG TGATGCGAGC GCGGTGGAAA TTAAATTTAG CAACGGCTCT
421 CAGGACATTC TGCTGCCGAA CGTGATTATT ATGGGCGCGG AACCGGACCT GTTTGAAACC
481 AACAGCAGCA ACATTAGCCT GCGTAACAAC TATATGCCGA GCAACCATGG TTTTGGCAGC
541 ATTGCGATTG TGACCTTTAG CCCGGAATAT AGCTTTCGCT TCAACGATAA CAGCATGAAC
601 GAATTTATTC AGGACCCGGC GCTGACCCTG ATGCACGAAC TGATTCATAG CCTGCATGGC
661 CTGTATGGCG CGAAAGGCAT TACCACCAAA TATACCATCA CCCAGAAACA GAATCCGCTG
721 ATTACCAACA TTCGTGGCAC CAACATTGAA GAATTTCTGA CCTTTGGCGG CACCGATCTG
781 AACATTATTA CCAGCGCGCA GAGCAACGAT ATCTATACCA ACCTGCTGGC CGATTATAAA
841 AAAATCGCGT CTAAACTGAG CAAAGTGCAG GTGAGCAATC CGCTGCTGAA TCCGTATAAA
901 GATGTGTTTG AAGCGAAATA TGGCCTGGAT AAAGATGCTA GCGGCATTTA TAGCGTGAAC
961 ATCAACAAAT TCAACGACAT CTTCAAAAAA CTGTATAGCT TTACCGAATT TGATCTGGCC
 
1021 ACCAAATTTC AGGTGAAATG CCGCCAGACC TATATTGGCC AGTATAAATA TTTTAAACTG
1081 AGCAACCTGC TGAACGATAG CATTTACAAC ATCAGCGAAG GCTATAACAT CAACAACCTG
1141 AAAGTGAACT TTCGTGGCCA GAACGCGAAT TTAAATCCGC GTATTATTAC CCCGATTACC
1201 GGCCGTGGAC TAGTGAAAAA AATTATCCGT TTTTGCGTGC GTGGCATTAT CACCAGCAAA
1261 ACCAAAAGCC TGGTGCCGCG TGGCAGCAAA GCGTTAAATG ATTTATGCAT CGAAATCAAC
1321 AACGGCGAAC TGTTTTTTGT GGCGAGCGAA AACAGCTATA ACGATGATAA CATCAACACC
1381 CCGAAAGAAA TTGATGATAC CGTGACCAGC AATAACAACT ACGAAAACGA TCTGGATCAG
1441 GTGATTCTGA ACTTTAACAG CGAAAGCGCA CCGGGCCTGT CTGATGAAAA ACTGAACCTG
1501 ACCATTCAGA ACGATGCGTA TATCCCGAAA TATGATAGCA ACGGCACCAG CGATATTGAA
1561 CAGCATGATG TGAACGAACT GAACGTGTTT TTTTATCTGG ATGCGCAGAA AGTGCCGGAA
1621 GGCGAAAACA ACGTGAATCT GACCAGCTCA ATTGATACCG CGCTGCTGGA ACAGCCGAAA
1681 ATCTATACCT TTTTTAGCAG CGAATTCATC AACAACGTGA ACAAACCGGT GCAGGCGGCG
1741 CTGTTTGTGA GCTGGATTCA GCAGGTGCTG GTTGATTTTA CCACCGAAGC GAACCAGAAA
1801 AGCACCGTGG ATAAAATTGC GGATATTAGC ATTGTGGTGC CGTATATTGG CCTGGCCCTG
1861 AACATTGGCA ACGAAGCGCA GAAAGGCAAC TTTAAAGATG CGCTGGAACT GCTGGGTGCG
1921 GGCATTCTGC TGGAATTTGA ACCGGAACTG CTGATTCCGA CCATTCTGGT GTTTACCATC
1981 AAAAGCTTTC TGGGCAGCAG CGATAACAAA AACAAAGTGA TCAAAGCGAT TAACAACGCG
2041 CTGAAAGAAC GTGATGAAAA ATGGAAAGAA GTGTATAGCT TCATTGTGTC TAACTGGATG
2101 ACCAAAATCA ACACCCAGTT CAACAAACGT AAAGAACAAA TGTATCAGGC GCTGCAGAAC
2161 CAGGTGAACG CGATTAAAAC CATCATCGAA AGCAAATACA ACAGCTACAC CCTGGAAGAA
2221 AAAAACGAAC TGACCAACAA ATATGACATC AAACAAATCG AAAATGAACT GAACCAGAAA
2281 GTGAGCATTG CCATGAACAA CATTGATCGC TTTCTGACCG AAAGCAGCAT TAGCTACCTG
2341 ATGAAACTGA TCAACGAAGT GAAAATCAAC AAACTGCGCG AATATGATGA AAACGTGAAA
2401 ACCTACCTGC TGAACTATAT TATTCAGCAT GGCAGCATTC TGGGCGAAAG CCAGCAAGAA
2461 CTGAACAGCA TGGTTACCGA TACCCTGAAC AACAGCATTC CGTTTAAACT GAGCAGCTAC
2521 ACCGATGATA AAATCCTGAT CAGCTACTTC AACAAATTCT TCAAACGCAT CAAAAGCAGC
2581 AGCGTGCTGA ACATGCGTTA TAAAAACGAT AAATACGTAG ATACCAGCGG CTATGATAGC
2641 AATATCAACA TTAACGGTGA TGTGTATAAA TACCCGACCA ACAAAAACCA GTTCGGCATC
2701 TACAACGATA AACTGAGCGA AGTGAACATT AGCCAGAACG ATTATATCAT CTACGATAAT
2761 AAATATAAAA ACTTCAGCAT CAGCTTTTGG GTGCGTATTC CGAACTACGA TAACAAAATC
2821 GTGAACGTGA ACAACGAATA CACCATCATT AACTGCATGC GTGATAACAA CAGCGGCTGG
2881 AAAGTGAGCC TGAACCATAA CGAAATCATC TGGACCCTGC AGGATAACGC CGGCATTAAC
2941 CAGAAACTGG CCTTTAACTA TGGCAACGCG AACGGCATTA GCGATTACAT CAACAAATGG
3001 ATCTTTGTGA CCATTACCAA CGATCGTCTG GGCGATAGCA AACTGTATAT TAACGGCAAC
3061 CTGATCGACC AGAAAAGCAT TCTGAACCTG GGCAACATTC ATGTGAGCGA TAACATCCTG
3121 TTCAAAATTG TGAACTGCAG CTATACCCGT TATATTGGCA TCCGCTATTT CAACATCTTC
3181 GATAAAGAAC TGGATGAAAC CGAAATTCAG ACCCTGTATA GCAACGAACC GAACACCAAC
3241 ATCCTGAAAG ATTTCTGGGG CAACTATCTG CTGTACGATA AAGAATATTA TCTGCTGAAC
3301 GTGCTGAAAC CGAACAACTT TATTGATCGC CGTAAAGATA GCACCCTGAG CATTAACAAC
3361 ATTCGTAGCA CCATTCTGCT GGCCAACCGT CTGTATAGCG GCATTAAAGT GAAAATTCAG
3421 CGCGTGAACA ATAGCAGCAC CAACGATAAC CTGGTGCGTA AAAACGATCA GGTGTATATC
3481 AACTTTGTGG CCAGCAAAAC CCACCTGTTT CCGCTGTATG CGGATACCGC GACCACCAAC
3541 AAAGAAAAAA CCATTAAAAT CAGCAGCAGC GGCAACCGTT TTAACCAGGT GGTGGTGATG
3601 AACAGCGTGG GCAACAACTG TACAATGAAC TTCAAAAACA ACAACGGCAA CAACATTGGC
3661 CTGCTGGGCT TTAAAGCGGA TACCGTGGTG GCGAGCACCT GGTATTATAC CCACATGCGT
3721 GATCATACCA ACAGCAACGG CTGCTTTTGG AACTTTATTA GCGAAGAACA TGGCTGGCAG
3781 GAAAAATAA
**SEQ ID NO: 6:**

1 MPKINSFNYN DPVNDRTILY IKPGGCQEFY KSFNIMKNIW IIPERNVIGT
51 TPQDFHPPTS LKNGDSSYYD PNYLQSDEEK DRFLKIVTKI FNRINNNLSG
101 GILLEELSKA NPYLGNDNTP DNQFHIGDAS AVEIKFSNGS QDILLPNVII
151 MGAEPDLFET NSSNISLRNN YMPSNHGFGS IAIVTFSPEY SFRFNDNSMN
201 EFIQDPALTL MHELIHSLHG LYGAKGITTK YTITQKQNPL ITNIRGTNIE

251 EFLTFGGTDL NIITSAQSND IYTNLLADYK KIASKLSKVQ VSNPLLNPYK
301 DVFEAKYGLD KDASGIYSVN INKFNDIFKK LYSFTEFDLA TKFQVKCRQT
351 YIGQYKYFKL SNLLNDSIYN ISEGYNINNL KVNFRGQNAN LNPRIITPIT
401 GRGLVKKIIR FCVRGIITSK TKSLVPR*GSKALNDLCIEIN NGELFFVASE
451 NSYNDDNINT PKEIDDTVTS NNNYENDLDQ VILNFNSESA PGLSDEKLNL
501 TIQNDAYIPK YDSNGTSDIE QHDVNELNVF FYLDAQKVPE GENNVNLTSS
551 IDTALLEQPK IYTFFSSEFI NNVNKPVQAA LFVSWIQQVL VDFTTEANQK
601 STVDKIADIS IVVPYIGLAL NIGNEAQKGN FKDALELLGA GILLEFEPEL
651 LIPTILVFTI KSFLGSSDNK NKVIKAINNA LKERDEKWKE VYSFIVSNWM
701 TKINTQFNKR KEQMYQALQN QVNAIKTIIE SKYNSYTLEE KNELTNKYDI
751 KQIENELNQK VSIAMNNIDR FLTESSISYL MKLINEVKIN KLREYDENVK
801 TYLLNYIIQH GSILGESQQE LNSMVTDTLN NSIPFKLSSY TDDKILISYF
851 NKFFKRIKSS SVLNMRYKND KYVDTSGYDS NININGDVYK YPTNKNQFGI
901 YNDKLSEVNI SQNDYIIYDN KYKNFSISFW VRIPNYDNKI VNVNNEYTII
951 NCMRDNNSGW KVSLNHNEII WTLQDNAGIN QKLAFNYGNA NGISDYINKW
1001 IFVTITNDRL GDSKLYINGN LIDQKSILNL GNIHVSDNIL FKIVNCSYTR
1051 YIGIRYFNIF DKELDETEIQ TLYSNEPNTN ILKDFWGNYL LYDKEYYLLN
1101 VLKPNNFIDR RKDSTLSINN IRSTILLANR LYSGIKVKIQ RVNNSSTNDN
1151 LVRKNDQVYI NFVASKTHLF PLYADTATTN KEKTIKISSS GNRFNQVVVM
1201 NSVGNNCTMN FKNNNGNNIG LLGFKADTVV ASTWYYTHMR DHTNSNGCFW
1251 NFISEEHGWQ EK

### Comparative Example 2: Cleavage of single-chain botulinum neurotoxin precursor molecule using human thrombin

The product of example 1 in phosphate buffer pH 7.8 and 150 mM NaCl is treated for 90 min with 25 IU (international units) human thrombin (Recothrom^{®}) per mg BoNT/E at 4-8°C. Thereafter, the amount of cleavage to the di-chain molecule is evaluated by evaluate expression by SDS polyacrylamide gel electrophoresis of the reaction under both reducing and non-reducing conditions, followed by Western blot analysis using anti-BoNT/E antibodies. The disulfide-bonded heterodimeric BoNT/E molecule applied onto SDS-PAGE under non-reducing conditions results in a protein band of approx. 150 kDa, while the same sample applied under reducing conditions results in two proteins of 100 kDa and 50 kDa, corresponding to the heavy and light chain, respectively.

### Comparative Example 3: Cleavage of single-chain botulinum neurotoxin precursor molecule using human factor Xa

Purified single-chain Botulinum neurotoxin type E (30 µg) generated and expressed in accordance with the protocol of Example 1 was treated and incubated with 40 IU (international units) human factor Xa (Novagen, Cat. No. 69036-3, Lot D00134035) for 24 h at 20°C in phosphate buffer pH 7.8 and 150 mM NaCl. Thereafter, it could be shown by SDS-PAGE that a two-chain molecule had been formed. Examination by nano-CL-ESI-FT MS/MS showed that the C-terminus of the light chain ended in the sequence ...LVPR, while Edman degradation of the heavy chain identified an N-terminus having the sequence GS.... . Thus, it could be shown that factor Xa is cleaving the single-chain precursor molecule C-terminally of the arginine residue in the thrombin recognition sequence.

## Claims

1. A method for the generation of a disulfide-linked di-chain clostridial neurotoxin, comprising the step of
(i) treating a disulfide-linked single-chain precursor clostridial neurotoxin molecule, which comprises a cleavage signal for human thrombin in the loop region linking the C-terminus of the light chain with the N-terminus of the heavy chain, wherein the loop region linking the C-terminus of the light chain with the N-terminus of the heavy chain of the clostridial neurotoxin comprises the polypeptide motif ENKSLVPRGS, with human thrombin.

2. The method of claim 1, wherein the human thrombin is a human thrombin-containing drug product.

3. The method of claim 1 or 2, wherein the human thrombin is a recombinant human thrombin.

4. The method of any one of claim 1 to 3, wherein said step (i) is performed in a buffer solution selected from the list of:
(i) Tris pH 7.6-8.0 and 70-150 mM NaCl;
(ii) Tris pH 7.6-8.0 and 300-500 mM NaCl;
(iii) Tris pH 7.8-8.2 and 20 mM NaCl;
(iv) Phosphate buffer pH 7.6-8.0 and 70-150 mM NaCl;
(v) Phosphate buffer pH 7.6-8.0 and 300-500 mM NaCl;
(vi) Phosphate buffer pH 7.6-8.0 and 20 mM NaCl;
(vii) HEPES pH 7.6-8.0 and 70-150 mM NaCl;
(viii) HEPES pH 7.6-8.0 and 300-500 mM NaCl; and
(ix) HEPES pH 7.6-8.2 and 20 mM NaCl.

5. The method of any one of claim 1 to 4, wherein the clostridial neurotoxin is selected from Clostridium botulinum neurotoxin serotype A, B, C, D, E, F, and G, particularly Clostridium botulinum neurotoxin serotype A and E, particularly Clostridium botulinum neurotoxin serotype E, more particularly a modified Clostridium botulinum neurotoxin serotype E.

6. The method of any one of claims 1 to 5, further comprising the step of
(ii) isolating the disulfide-linked di-chain clostridial neurotoxin by chromatography on an ion exchange matrix or a hydrophobic interaction matrix or a multimodal chromatography matrix, particularly a strong ion exchange matrix, more particularly a strong cation exchange matrix.

7. The method of claim 6, wherein step (ii) comprises the step(s) of:
(iia) a conditioning step to obtain a low-salt solution at a pH of between about 7.9 and about 8.1;
(iib) application of said low-salt solution containing the disulfide-linked di-chain clostridial neurotoxin on a sulfopropyl-substituted chromatography matrix;
(iic) washing the sulfopropyl-substituted chromatography matrix with a buffer solution at a pH of between about 7.9 and about 8.1 and containing about 20 mM salt; and
(iid) elution of the disulfide-linked di-chain clostridial neurotoxin from the sulfopropyl-substituted chromatography matrix by increasing the salt concentration of the buffer solution of step (iic) to between about 50 and about 500 mM salt.

8. A single-chain precursor clostridial neurotoxin molecule comprising (i) a functionally active clostridial neurotoxin light chain, (ii) a functionally active clostridial neurotoxin heavy chain, and (iii) a loop region linking the C-terminus of the light chain with the N-terminus of the heavy chain comprises a cleavage signal for human thrombin, wherein the loop region linking the C-terminus of the light chain with the N-terminus of the heavy chain of the clostridial neurotoxin comprises the polypeptide motif ENKSLVPRGS.

9. A nucleic acid sequence encoding the single-chain precursor clostridial neurotoxin molecule of claim 8.

10. A method for obtaining the nucleic acid sequence of claim 9, comprising the step of inserting a cleavage signal for human thrombin into a nucleic acid sequence encoding a single-chain precursor clostridial neurotoxin molecule.

11. A vector comprising the nucleic acid sequence of claim 9, or the nucleic acid obtainable by the method of claim 10.

12. A recombinant host cell comprising the nucleic acid sequence of claim 9, or the vector of claim 11.

13. A method for producing the single-chain precursor clostridial neurotoxin molecule of claim 8, comprising the step of expressing the nucleic acid sequence of claim 9, the nucleic acid sequence obtainable by the method of claim 10, or the vector of claim 11 in a recombinant host cell, or cultivating the recombinant host cell of claim 12 under conditions that result in the expression of said nucleic acid sequence.

14. A disulfide-linked di-chain clostridial neurotoxin, wherein the C-terminus of the light chain ends with the sequence KSLVPR, and the N-terminus of the heavy chain begins with the sequence GSK.

15. A pharmaceutical composition comprising the disulfide-linked di-chain clostridial neurotoxin of claim 14.

## Patentansprüche

1. Verfahren zur Erzeugung eines Disulfid-verknüpften zweikettigen Clostridium-Neurotoxins, umfassend den folgenden Schritt:
(i) Behandeln eines Disulfid-verknüpften einkettigen Vorläufer-Clostridium-Neurotoxin-Moleküls, das ein Spaltsignal für menschliches Thrombin in dem Schleifenbereich umfasst, das den C-Terminus der leichten Kette mit dem N-Terminus der schweren Kette verbindet, wobei die Schleifenregion, die den C-Terminus der leichten Kette mit dem N-Terminus der schweren Kette des Clostridium-Neurotoxins verbindet, das Polypeptidmotiv ENKSLVPRGS mit menschlichem Thrombin umfasst.

2. Verfahren nach Anspruch 1, wobei das menschliche Thrombin ein menschliches Thrombin-haltiges Arzneimittel ist.

3. Verfahren nach Anspruch 1 oder 2, wobei das menschliche Thrombin ein rekombinantes menschliches Thrombin ist.

4. Verfahren nach einem der Ansprüche 1 bis 3, wobei der Schritt (i) in einer Pufferlösung durchgeführt wird, die aus der Liste ausgewählt ist:
(i) Tris pH 7,6-8,0 und 70-150 mM NaCl;
(ii) Tris pH 7,6-8,0 und 300-500 mM NaCl;
(iii) Tris pH 7,8-8,2 und 20 mM NaCl;
(iv) Phosphatpuffer pH 7,6-8,0 und 70-150 mM NaCl;
(v) Phosphatpuffer pH 7,6-8,0 und 300-500 mM NaCl;
(vi) Phosphatpuffer pH 7,6-8,0 und 20 mM NaCl;
(vii) HEPES pH 7,6-8,0 und 70-150 mM NaCl;
(viii) HEPES pH 7,6-8,0 und 300-500 mM NaCl; und
(ix) HEPES pH 7,6-8,2 und 20 mM NaCl.

5. Verfahren nach einem der Ansprüche 1 bis 4, wobei das Clostridium-Neurotoxin aus Clostridium botulinum Neurotoxin Serotyp A, B, C, D, E, F und G, insbesondere Clostridium botulinum Neurotoxin Serotyp A und E, insbesondere Clostridium Botulinum-Neurotoxin-Serotyp E ausgewählt ist, und insbesondere ein modifizierter Clostridium-Botulinum-Neurotoxin-Serotyp E ist.

6. Verfahren nach einem der Ansprüche 1 bis 5, weiter umfassend den Schritt
(ii) Isolieren des disulfidverknüpften zweikettigen Clostridium-Neurotoxins durch Chromatographie an einer lonenaustauschermatrix oder einer hydrophoben Interaktionsmatrix oder einer multimodalen Chromatographiematrix, insbesondere einer starken lonenaustauschermatrix, insbesondere einer starken Kationenaustauschermatrix.

7. Verfahren nach Anspruch 6, wobei Schritt (ii) die folgenden Schritte umfasst:
(iia) einen Konditionierungsschritt, um eine salzarme Lösung bei einem pH-Wert zwischen etwa 7,9 und etwa 8,1 zu erhalten;
(iib) Anwenden der Lösung mit niedrigem Salzgehalt, die das Disulfid-verknüpfte zweikettige Clostridium-Neurotoxin enthält, auf einer Sulfopropyl-substituierten Chromatographiematrix;
(iic) Waschen der Sulfopropyl-substituierten Chromatographiematrix mit einer Pufferlösung bei einem pH-Wert zwischen etwa 7,9 und etwa 8,1 und enthaltend etwa 20 mM Salz; und
(iid) Elution des disulfidverknüpften zweikettigen-Clostridium-Neurotoxins aus der Sulfopropyl-substituierten Chromatographiematrix durch Erhöhung der Salzkonzentration der Pufferlösung von Schritt (iic) auf zwischen etwa 50 und etwa 500 mM Salz.

8. Einkettiges Vorstufen-Clostridium-Neurotoxin-Molekül, umfassend (i) eine funktionell aktive Clostridium-Neurotoxin-Leichtkette, (ii) eine funktionell aktive Clostridium-Neurotoxin-Schwerkette und (iii) einen Schleifenbereich, der den C-Terminus der leichten Kette mit dem N-Terminus der schweren Kette verbindet, umfassend ein Spaltsignal für humanes Thrombin, wobei der Schleifenbereich, der den C-Terminus der leichten Kette mit dem N-Terminus der schweren Kette des Clostridium-Neurotoxins verbindet, das Polypeptidmotiv ENKSLVPRGS umfasst.

9. Nukleinsäuresequenz, die für das einkettige Vorläufer-Clostridium-Neurotoxinmolekül nach Anspruch 8 kodiert.

10. Verfahren zur Herstellung der Nukleinsäuresequenz nach Anspruch 9, umfassend den Schritt des Einfügens eines Spaltsignals für menschliches Thrombin in eine Nukleinsäuresequenz, die für ein einkettiges Vorläufer-Clostridium-Neurotoxinmolekül kodiert.

11. Vektor, umfassend die Nukleinsäuresequenz nach Anspruch 9 oder die nach dem Verfahren nach Anspruch 10 erhältliche Nukleinsäure.

12. Rekombinante Wirtszelle, umfassend die Nukleinsäuresequenz nach Anspruch 9 oder den Vektor nach Anspruch 11.

13. Verfahren zur Herstellung des einkettigen Vorläufer-Clostridium-Neurotoxinmoleküls nach Anspruch 8, umfassend den Schritt des Exprimierens der Nukleinsäuresequenz nach Anspruch 9, die nach dem Verfahren des Anspruchs 10 erhältliche Nukleinsäuresequenz oder des Vektors nach Anspruch 11 in eine rekombinante Wirtszelle, oder Kultivieren der rekombinanten Wirtszelle nach Anspruch 12 unter Bedingungen, die zur Expression der Nukleinsäuresequenz führen.

14. Disulfid-verknüpftes zweikettiges Clostridium-Neurotoxin, wobei der C-Terminus der leichten Kette mit der Sequenz KSLVPR endet, und der N-Terminus der schweren Kette mit der Sequenz GSK beginnt.

15. Pharmazeutische Zusammensetzung, umfassend das Disulfid-verknüpfte zweikettige Clostridium-Neurotoxin nach Anspruch 14.

## Revendications

1. Procédé pour la génération d'une neurotoxine clostridienne à deux chaînes et à liaison disulfure, comprenant l'étape suivante
(i) traiter une molécule de neurotoxine clostridienne précurseur à une seule chaîne et à liaison disulfure, qui comprend un signal de coupure pour la thrombine humaine dans la région de boucle reliant l'extrémité C de la chaîne légère à l'extrémité N de la chaîne lourde, la région de boucle reliant l'extrémité C de la chaîne légère à l'extrémité N de la chaîne lourde de la neurotoxine clostridienne comprenant le motif polypeptidique ENKSLVPRGS, avec de la thrombine humaine.

2. Procédé selon la revendication 1, dans lequel la thrombine humaine est un produit médicamenteux contenant de la thrombine humaine.

3. Procédé selon la revendication 1 ou 2, dans lequel la thrombine humaine est une thrombine humaine recombinée.

4. Procédé selon l'une quelconque des revendications 1 à 3, dans lequel ladite étape (i) est effectuée dans une solution tampon choisie dans la liste suivante :
(i) Tris pH 7,6-8,0 et NaCl 70-150 mM ;
(ii) Tris pH 7,6-8,0 et NaCl 300-500 mM ;
(iii) Tris pH 7,8-8,2 et NaCl 20 mM ;
(iv) tampon phosphate pH 7,6-8,0 et NaCl 70-150 mM ;
(v) tampon phosphate pH 7,6-8,0 et NaCl 300-500 mM ;
(vi) tampon phosphate pH 7,6-8,0 et NaCl 20 mM ;
(vii) HEPES pH 7,6-8,0 et NaCl 70-150 mM ;
(viii) HEPES pH 7,6-8,0 et NaCl 300-500 mM ; et
(ix) HEPES pH 7,6-8,2 et NaCl 20 mM.

5. Procédé selon l'une quelconque des revendications 1 à 4, dans lequel la neurotoxine clostridienne est choisie parmi les sérotypes A, B, C, D, E, F et G de neurotoxine de Clostridium botulinum, en particulier les sérotypes A et E de neurotoxine de Clostridium botulinum, en particulier un sérotype E de neurotoxine de Clostridium botulinum, plus particulièrement un sérotype E de neurotoxine de Clostridium botulinum modifié.

6. Procédé selon l'une quelconque des revendications 1 à 5, comprenant en outre l'étape suivante
(ii) isoler la neurotoxine clostridienne à deux chaînes et à liaison disulfure par chromatographie sur une matrice échangeuse d'ions ou une matrice à interaction hydrophobe ou une matrice de chromatographie multimodale, en particulier une matrice fortement échangeuse d'ions, plus particulièrement une matrice fortement échangeuse de cations.

7. Procédé selon la revendication 6, dans lequel l'étape (ii) comprend la ou les étapes suivante :
(iia) une étape de conditionnement pour que soit obtenue une solution à faible teneur en sel à un pH compris entre environ 7,9 et environ 8,1 ;
(iib) l'application de ladite solution à faible teneur en sel contenant la neurotoxine clostridienne à deux chaînes et à liaison disulfure sur une matrice de chromatographie à substitution sulfopropyle ;
(iic) le lavage de la matrice de chromatographie à substitution sulfopropyle avec une solution tampon à un pH compris entre environ 7,9 et environ 8,1 et contenant du sel à environ 20 mM ; et
(iid) l'élution de la neurotoxine clostridienne à deux chaînes et à liaison disulfure à partir de la matrice de chromatographie à substitution sulfopropyle par augmentation de la concentration de sel de la solution tampon de l'étape (iic) à une concentration de sel comprise entre environ 50 et environ 500 mM.

8. Molécule de neurotoxine clostridienne précurseur à une seule chaîne comprenant (i) une chaîne légère de neurotoxine clostridienne fonctionnellement active, (ii) une chaîne lourde de neurotoxine clostridienne fonctionnellement active, et (iii) une région de boucle reliant l'extrémité C de la chaîne légère à l'extrémité N de la chaîne lourde, qui comprend un signal de coupure pour la thrombine humaine, dans laquelle la région de boucle reliant l'extrémité C de la chaîne légère à l'extrémité N de la chaîne lourde de la neurotoxine clostridienne comprend le motif polypeptidique ENKSLVPRGS.

9. Séquence d'acide nucléique codant la molécule de neurotoxine clostridienne précurseur à une seule chaîne de la revendication 8.

10. Procédé pour obtenir la séquence d'acide nucléique de la revendication 9, comprenant l'étape d'insertion d'un signal de coupure pour la thrombine humaine dans une séquence d'acide nucléique codant une molécule de neurotoxine clostridienne précurseur à une seule chaîne.

11. Vecteur comprenant la séquence d'acide nucléique de la revendication 9, ou l'acide nucléique pouvant être obtenu par le procédé de la revendication 10.

12. Cellule hôte recombinée comprenant la séquence d'acide nucléique de la revendication 9, ou le vecteur de la revendication 11.

13. Procédé pour produire la molécule de neurotoxine clostridienne précurseur à une seule chaîne de la revendication 8, comprenant l'étape d'expression de la séquence d'acide nucléique de la revendication 9, la séquence d'acide nucléique pouvant être obtenue par le procédé de la revendication 10, ou le vecteur de la revendication 11, dans une cellule hôte recombinée, ou de culture de la cellule hôte recombinée de la revendication 12 dans des conditions ayant pour résultat l'expression de ladite séquence d'acide nucléique.

14. Neurotoxine clostridienne à deux chaînes et à liaison disulfure, dans laquelle l'extrémité C de la chaîne légère se termine par la séquence KSLVPR, et l'extrémité N de la chaîne lourde commence par la séquence GSK.

15. Composition pharmaceutique comprenant la neurotoxine clostridienne à deux chaînes et à liaison disulfure de la revendication 14.
